# EUROPEAN PATENT APPLICATION

(11) **EP 1 308 542 A2**
(43) Date of publication of application: **07.05.2003**
(21) Application number: 02256721.8
(22) Date of filing: 26.09.2002
(51) Int. Cl.: C30B 7/00

(54) **Method and apparatus for crystallization of proteins and the like**

(30) Priority: 27.09.2001 JP 2001298140
(71) Applicant: Riken, Wako-shi, Saitama 351-0198 (JP); Advansoft Inc., Suita-shi, Osaka 564-0052 (JP); Stec Co., Ltd, Yatsuka-gun, Shimane 699-0101 (JP); Takeda Rika Kogyo Co. Ltd, Shibuya-ku, Tokyo 150-0021 (JP)
(72) Inventor: Sugahara, Mitsuaki c/o Riken Harima Institute, Hyogo 679-5148 (JP); Miyano, Masashi c/o Riken Harima Institute, Hyogo 679-5148 (JP); Nishio, Kazuya c/o Riken Harima Institute, Hyogo 679-5148 (JP); Nagashima, Masatsugu c/o Stec Co., Ltd., Yatsuka-gun Shimane 699-0101 (JP); Yamai, Shigeo, Osaka 562-0046 (JP); Kawamura, Kiyokazu, Kobe-shi, Hyogo 651-1121 (JP)
(74) Representative: Lord, Hilton David

(57) **Abstract**

In the method of the invention for crystallization of proteins and the like, test substances prepared by mixing a sample of a protein and the like and reagents which promote the crystallization of the sample are put into test-substance wells of containers, such as microplates, and transferred to an analysis process, such as an X-ray crystal structural analysis, to perform an information analysis of the sample. This method includes the steps of: setting a plurality of standard images of progress stages of the test substances, such as crystal formation, as images in uncompleted crystallization regions and images in crystallization regions permitting an analysis; comparing data on the plurality of standard images with fetched image data during the progress of crystallization of the test substances thereby to make a judgment; selecting only test substances having fetched image data in perfect or close agreement with the images in crystallization regions permitting an analysis; and transferring a container containing the test substances to the analysis process.

In the above-described method, a mark to indicate the selection is automatically put to the container containing the test substances which are selected by comparing data on the plurality of standard images with the fetched image data to make a judgment.

The method of the invention includes a crystallization robot, which prepares test substances by mixing a sample of a protein and the like and reagents which promote the crystallization of the sample in test-substance wells of containers such as microplates; a stocker housing portion, which includes a plurality of shelves which temporarily store the containers containing the test substances in such a manner as to permit the mounting and dismounting thereof; an image processor, which performs image processing of the test samples in the containers stored in the stocker housing portion thereby to grasp the condition of crystallization; a stocker robot, which performs the mounting and dismounting of the containers in the stocker housing portion; and a stage robot, which performs the transit of the containers from the stocker robot and the transfer and supply of the containers to the crystallization robot and the image processor.

## Description

### BACKGROUND OF THE INVENTION

### Field of the Invention:

The present invention relates to a method and an apparatus for crystallization of proteins and the like, in which samples of proteins and the like are prepared as test substances in containers, such as microplates, and transferred to an analysis process, such as an X-ray crystal structural analysis, where an information analysis of the samples is performed.

### Description of the Prior Art:

In general, in the structural genomics which handles a large variety of proteins coming from various species of organisms, crystallization means of proteins and analysis means of crystallized proteins, such as an X-ray crystal structural analysis, are used in the structural analysis and processing of protein samples which are as large as tens of thousands in the number of types per year. This operation is performed in the flow of production and crystallization of proteins, collection of X-ray diffraction data, structural analysis and information analysis by use of databases etc.

And in the crystallization of proteins suitable for such an information analysis as described above, 72-well microplates are used and a screening of several hundred to several thousand conditions for each sample is performed. In the past, the above-described screening of conditions was carried out by a microbatch process in such a manner that in each test-substance well of a microplate, a protein sample and various kinds of crystallization solutions (reagents) in an amount of about 500 nanoliters per well are mixed by a dispensing operation by use of a dispenser, and this microplate for samples is subjected to crystallization treatment under temperature-controlled condition in oil. The amount of this crystallization operation corresponds to about as large as 1000 microplates, for example, for 20 to 30 samples per month.

Furthermore, for the test substances in the above-described microplates, their images for each inspection-substance well are visually checked under a microscope, a judgment is made based on the personal views of an observer in accordance with standard criteria as to whether there are crystals permitting an analysis are present, and features such as a precipitation condition, and the result of this judgment is stored in a database and used.

In the above-described crystallization operation, the mixing of protein samples and solutions for crystallization in each test-substance well of a microplate is almost automatically carried out by the dispensing operation by use of a dispenser, whereas the storage of the microplates after dispensing is carried out by human operations. Therefore, the efficiency of transfer of a large number of microplates is low and the efficiency of processing, such as the registration of the names of test substances is also low. Furthermore, the above-described method has such disadvantages as described below. That is, the storage efficiency is low when the microplates are to be stored in such a manner as to ensure that they can be easily mounted and dismounted, the transfer efficiency is also low when the microplates are to be transferred from storage places to next processes such as an image analysis, and troubles are apt to occur due to wrong operations associated with the storage and transfer.

Furthermore, in the above-described judgment as to whether an analysis of crystals of test substances in microplates is possible, the test substances in the microplates are observed by operating a motor-driven XYZ stage of a microscope and the judgment is made by analyzing quantities of characteristics related to the shapes and morphologies of crystallization drops, measured values related to concentrations, etc. while adding various human judgments to data on observed images. For this reason, there are disadvantages that the judgement result is influenced by the personal views of an operator and that facts are misidentified or overlooked. At the same time, because the judgment operation requires skills and prudence, the image analysis operation etc. become inefficient, thus posing a problem.

Therefore, in order to perform efficiently and with high accuracy the dispensing operation for a large number of microplates, the observation operation of crystallization drops by an image processor and the control of the microplates during these operations, etc., it is necessary to robotize (automate) each operation and, at the same time, to build a system for integrated control of the whole database. In the invention, the solution of the above-described problems is aimed at from the two aspects of hardware and software by building a system that integrates the whole, such as a microplate transfer system, and building and improving databases for evaluating crystallization drops and crystals on the basis of the development of automatic microdispensing techniques suitable for various types of samples and reagents used in crystallization and automatic observation techniques of crystallization drops whose identification is difficult, such as precipitants, microcrystals and amorphous.

### SUMMARY OF THE INVENTION:

The principal object of a method and an apparatus for crystallization of proteins and the like of the invention is to ensure that by automatically performing a crystallization check operation of all test substances in a container by use of an image processor in comparison with conventional methods and apparatuses based on human judgments by use of a microscope, the crystallization check is accurately and rapidly performed and storage management for the crystallization of the test substances for a long time is accurately performed with less labor.

A secondary object is to ensure that, by automatically putting a mark to indicate the selection etc. to a container having test substances which are selected by comparing standard image data with fetched image data to make a judgment, operations are efficiently carried out by preventing wrong transfer of the container etc. owing to the put mark etc.

A further object of the invention is to ensure that, by storing the containers housing the test substances on shelves of a stocker housing portion in such a manner as to permit the mounting and dismounting thereof, taking out the stored containers one after another, and repeatedly performing a selection of test substances by use of an image processor, the crystallization check operation by the image processor is accurately performed by preventing overlooks even for a large number of containers which store test substances for a long time.

Similarly, the object of the apparatus of the invention is to ensure that the transfer operation of containers is smoothly performed by use of a stocker robot and a stage robot, and at the same time, to simplify the mechanical configuration of the apparatus while protecting test substances, thereby to promote the crystallization of the test substances and improve the processing accuracy of the test substances.

Another object of the apparatus of the invention is to ensure that, by constructing the apparatus in such a manner that a container containing test substances selected by the image processor for transfer to an analysis process is stored in the stocker housing portion, the selected test substances in the container are efficiently transferred to the analysis process, whereby the crystallization of the test substances is promoted in the taking-out waiting time.

Smooth transit of containers on a crystallization device is ensured and, at the same time, each individual operation of the stage robot and stocker robot is efficiently performed by utilizing the transit waiting time of both.

The transfer of containers between the crystallization robot and the stocker robot and between the stocker robot and the image processor is efficiently performed and, at the same time, the construction of the stocker robot is simplified.

In order to achieve the above-described objects, according to a first aspect of the invention, there is provided a method for crystallization of proteins and the like of the invention, in which test substances prepared by mixing a sample of a protein and the like and reagents which promote the crystallization of the sample are put into test-substancewells of containers, such as microplates, and transferred to an analysis process, such as an X-ray crystal structural analysis, to perform an information analysis of the sample, comprises the steps of: setting a plurality of standard images of progress stages of the test substances, such as crystal formation, as images in uncompleted crystallization regions and images in crystallization regions permitting an analysis; comparing data on the plurality of standard images with fetched image data during the progress of crystallization of the test substances thereby to make a judgment; selecting only test substances having fetched image data in perfect or close agreement with the images in crystallization regions permitting an analysis; and transferring a container having the test substances to the analysis process.

According to a second aspect of the invention, a mark to indicate the selection is automatically put to the container containing the test substances which are selected by comparing data on the plurality of standard images with the fetched image data to make a judgment.

According to a third aspect of the invention, the containers containing the test substances are stored on shelves constituting a stocker housing portion in such a manner as to permit the mounting and dismounting thereof and the stored containers are taken out one after another, and a selection of test substances is repeatedly performed by use of an image processor.

According to a fourth aspect of the invention, there is provided an apparatus for crystallization of proteins and the like, which comprises: a crystallization robot, which prepares test substances by mixing a sample of a protein and the like and reagents which promote the crystallization of the sample in test-substance wells of containers such as microplates; a stocker housing portion, which comprises a plurality of shelves which temporarily store the containers housing the test substances in such a manner as to permit the mounting and dismounting thereof; an image processor, which
performs image processing of the test samples in the containers stored in the stocker housing portion thereby to grasp the condition of crystallization; a stocker robot, which performs the mounting and dismounting of the containers in the stocker housing portion; and a stage robot, which performs the transit of the containers from the stocker robot and the transfer and supply of the containers to the image processor.

According to a fifth aspect of the invention, the apparatus is constructed in such a manner that the container containing test substances selected by the image processor for transfer to the analysis process is stored in the stocker housing portion.

According to a sixth aspect of the invention, between the stocker robot which performs the mounting and dismounting of the containers in the stocker housing portion and the stage robot is provided a transit portion where the containers are temporarily placed and through this transit portion the transit of the containers between the stocker robot and the stage robot is performed.

According to a seventh aspect of the invention, the apparatus is constructed in such a manner the stage robot performs the transit of the containers containing the test substances from the crystallization robot side to the stocker robot side.

### BRIEF DESCRIPTION OF THE DRAWINGS

FIG. 1 is a front view of a crystallization apparatus of the invention;
FIG. 2 is a plan view of the crystallization apparatus of the invention;
FIG. 3 is a side sectional view showing the construction of essential parts of the apparatus of the invention;
FIG. 4 is a front view showing the construction of a stocker housing portion of the apparatus of the invention;
FIG. 5 is a front view of a crystallization robot;
FIG. 6 is a plan view showing the construction of essential parts of the crystallization robot;
FIG. 7 is a side sectional view of the crystallization robot;
FIG. 8 is a side view of the crystallization robot;
FIG. 9 is a system diagram showing the crystallization method of the invention and the operation of a management system; and
FIG. 10 is an at-a-glance view of image standards for test substances in an image processor related to the invention.

### DETAILED DESCRIPTION OF THE PREFERRED EMBODIMENT

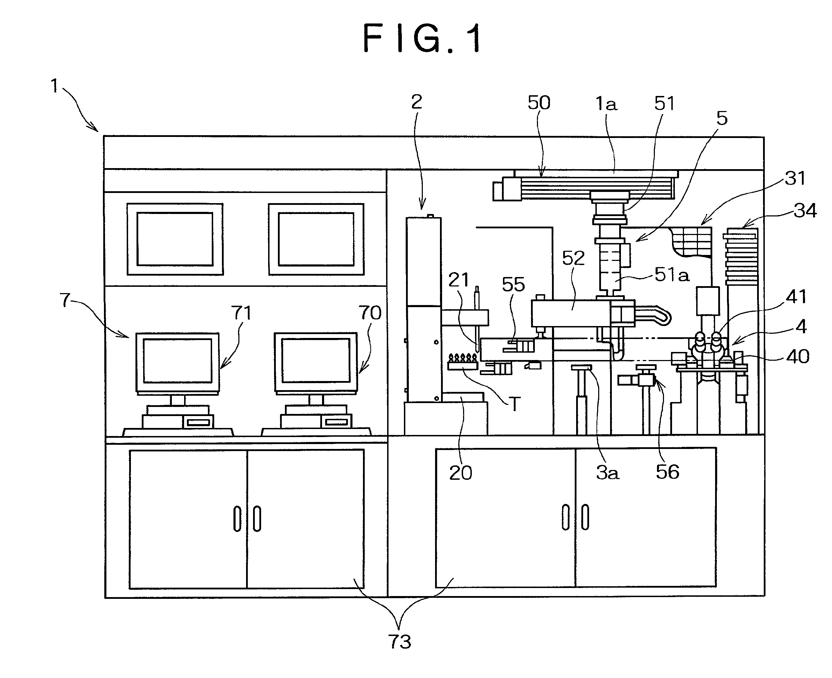

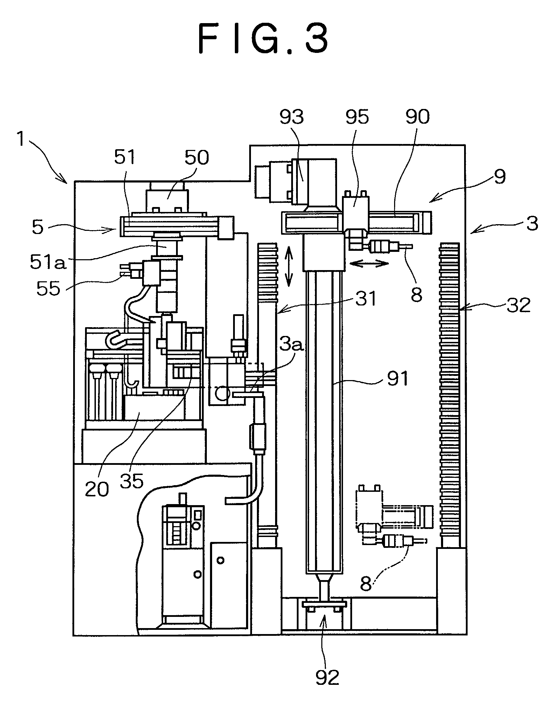

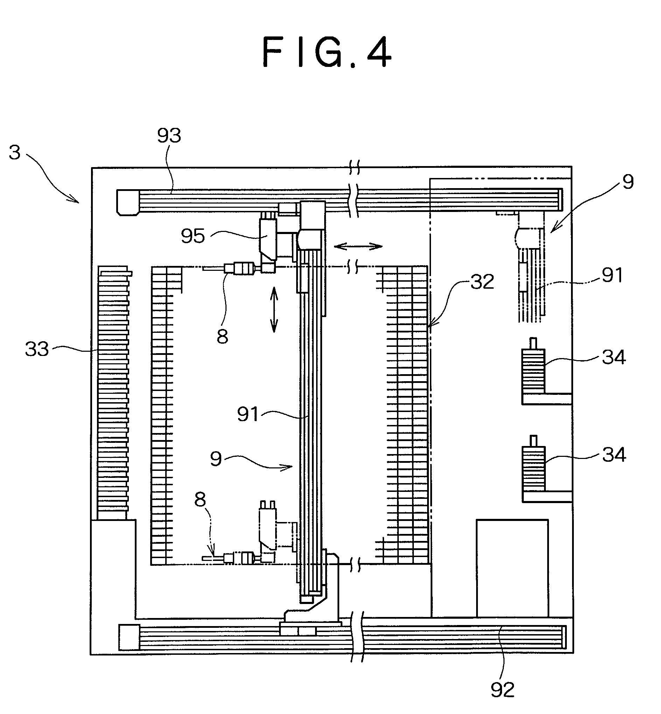

An embodiment of the invention will be described below on the basis of the drawings. In FIGS. 1 to 4, the reference numeral 1 denotes a management system (an automatic crystallization system) as a crystallization apparatus of proteins and the like, which comprises a system which can efficiently and very accurately perform the crystallization of various types of proteins (samples) by a crystallization method related to the invention, and the operations of storage, image processing, etc. of the proteins.

This management system 1 comprises: a crystallization robot 2, which prepares test substances by mixing various types of samples requiring a structural analysis and reagents, such as solutions, which promote the crystallization of the samples by a dispensing operation in test-substance wells m of microplates M as containers, which will be described later; a stocker housing portion 3, which comprises a plurality of shelves (stockers) 31, 32, 33 ..., which temporarily store the microplates M containing the test substances in such a manner as to permit the mounting and dismounting of the microplates M; an image processor 4, which performs the image processing of the test substances in the microplates M stored in the stocker housing portion 3 so as to permit visual judgment thereby to grasp the condition of crystallization; a stocker robot 9, which performs the mounting and dismounting of the microplates M in the stocker housing portion 3; a stage robot 5, which performs the transit and delivery/receiving of the microplates M and suspension plates S to the stocker robot 9, crystallization robot 2 and image processor 4 according to a set system procedure.

And in this management system 1, a transit portion 3a is formed as an entrance and exit of microplates M on the side of the stocker 31 of the stocker housing portion 3. Near or outside this transit portion 3a is provided an identification portion 6, which comprises a marking portion 60 which puts identification markings of microplates by barcodes, appropriate marks, etc. and a reading portion 61 which reads the put markings of the microplates M.

Furthermore, this management system comprises a control portion 7 which performs general control by means of a tracking control portion 70, which comprises a personal computer which performs the setting control of automatic operation procedures of the above-described parts and related equipment and adjustments and manual arbitrary operations, etc. of the above-described parts and related equipment, and a test-substance processing portion 71 which performs analysis, recording, etc. while performing the image display of images detected by the above-described image processor 4, data, etc . by means of a personal computer. The reference numeral 73 denotes a sequencer installation portion for each piece of equipment.

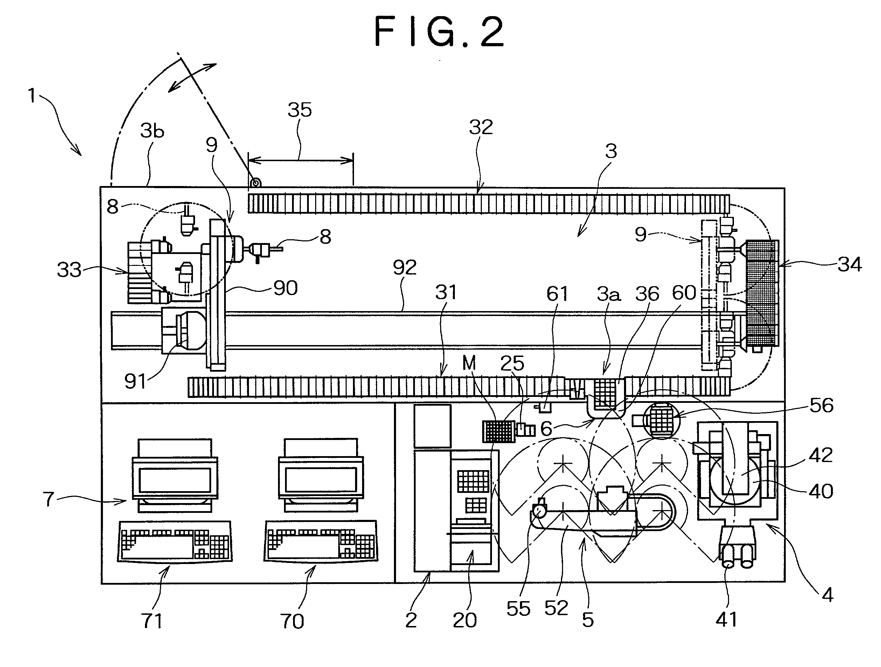

As shown in the plane view of FIG. 2, by adjacently installing the related devices and equipment with their mutual relations maintained, the devices and component portions of the invention are arranged in a united, compact manner without producing needless spaces in the general component space of the management system 1. And in this management system 1, by shortening the operating ranges of the microplates M etc. as far as possible and ensuring a speedy transfer operation of test substances, it is possible to simplify the devices and equipment while protecting the test substances, promote the crystallization of the test substances, and improve the processing accuracy of the test substances and general operation efficiency.

That is, in the illustrated management system 1, the stocker housing portion 3, which is configured so as to house a total of about 2500 microplates M on each shelf of a lateral rectangular shape in top and bottom stages, is horizontally installed, and in the space before this stocker housing portion 3 are arranged, from left, the above-described control portion 7, crystallization robot 2, stage robot 5, transit portion 3a and image processor 4 in this order. This enables these portions for the test-substance transfer operation to be adjacently disposed as a unity, and makes the shape of the management system 1 in the plan view laterally rectangular so that the management system can be installed in a building or a general temperature-controlled room in a space-saving manner.

Incidentally, the microplates M used in the management system 1 of this embodiment are generally-used plastics dish-shaped containers having 72 test-substance wells. The suspension plates S used are containers having 48 wells that can house various types of reagents in a solution state. The sample tubes T are 10 containers which house various types of protein samples. This construction aims at an improvement in the versatility, convenience and transfer efficiency of the management system 1.

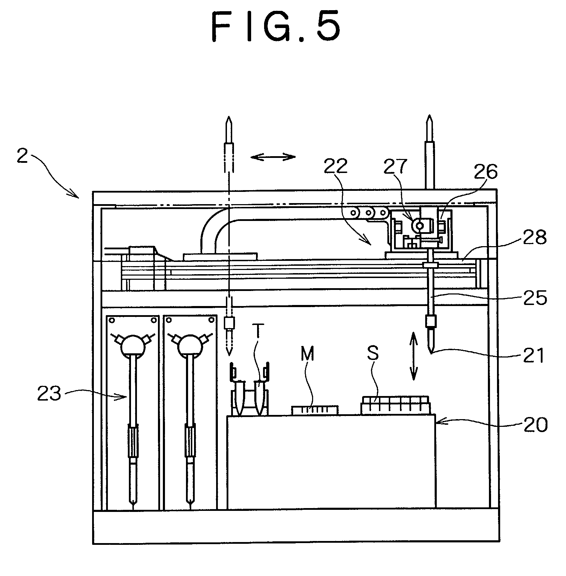

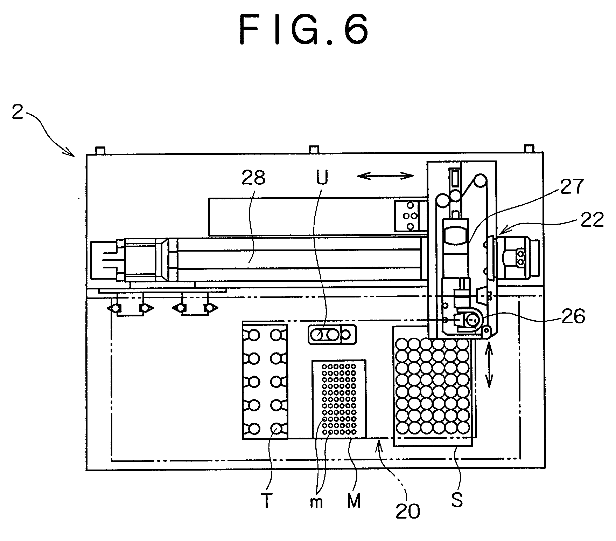

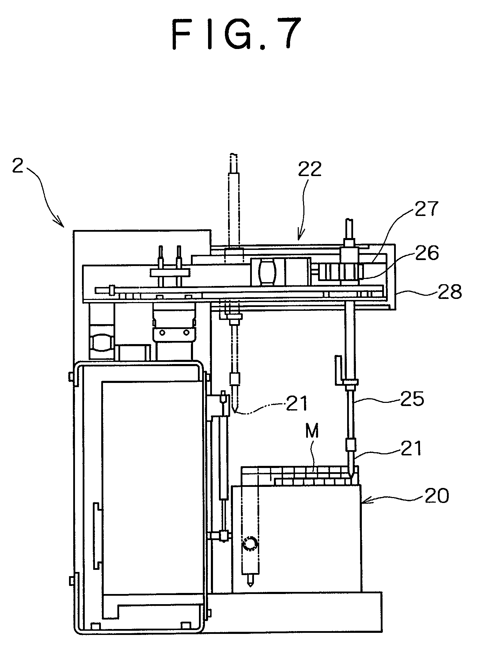

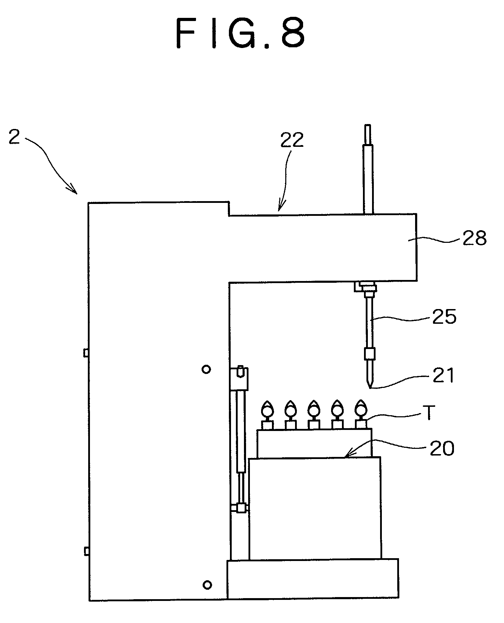

Next, the detailed construction of each part will be described below. First, as shown in FIG. 2, FIGS. 5 to 8, the crystallization robot 2 is provided with a plate bed 20 on which the sample tubes T, microplates M and suspension plates S are detachably placed in their respective prescribed positions on the side of the stage robot 5. Above this plate bed 20 is provided an actuating portion 22 which supports a dispensing tube 21 in such a manner that the dispensing tube can move in the X (transverse), Y (vertical) and Z (longitudinal) directions. Below this actuating portion 22 is provided a pump unit 23 which changeably gives a suction action and a delivery action to the dispensing tube 21.

Incidentally, a cleaning unit U installed on the plate bed 20 to clean the dispensing tube 21 and the interior of the dispensing tube 21 as required is the same known device as used in conventional dispensers.

The above-described actuating portion 22 will be described in detail. The illustrated actuating portion 22 is constituted by a Y-axis actuating portion 26, which vertically moves a lifting lever 25 provided with the dispensing tube 21 at its bottom end, an X-axis actuating portion 27, which laterally moves this Y-axis actuating portion 26, and a Z-axis actuating portion 28, which longitudinally moves an actuating arm supporting the X-axis actuating portion 27.

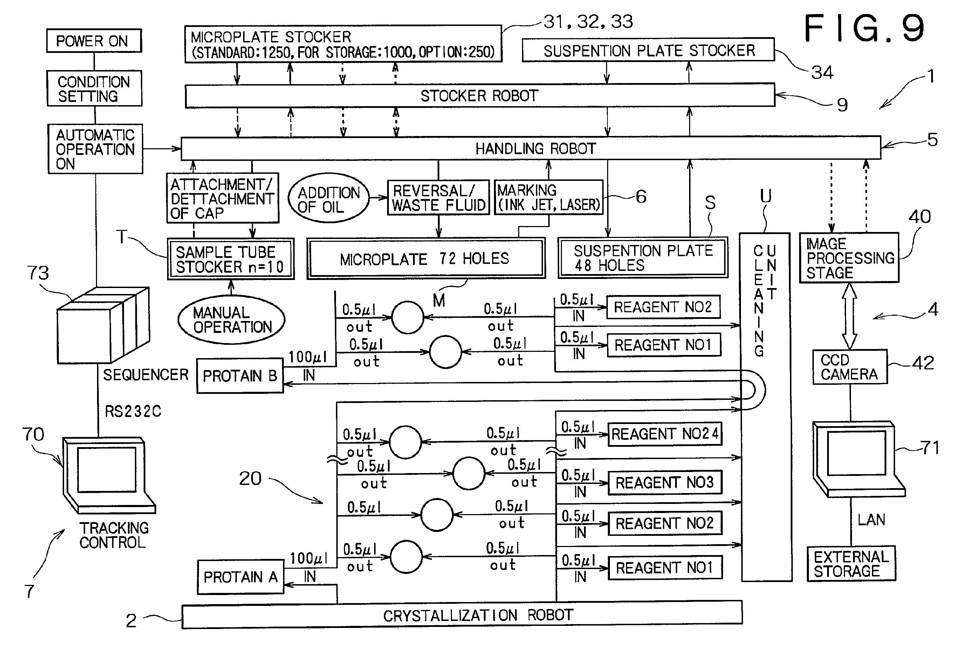

The dispensing tube 21 moves back and forth, from side to side, and up and down according to a dispensing operation program set by the control portion 7, and performs the dispensing operation to the microplates M with high accuracy in the mode shown in FIG. 9 by dispensing various types of samples (Protein A, Protein B, ...) in the sample tubes T and various types of solutions (Reagent No. 1, Reagent No. 2, ...) in their prescribed proportions.

Incidentally, in this dispensing operation, the dispensing tube 21 retracts to the cleaning unit U for each dispensing step and is automatically subjected to the cleaning of the interior of the conduit, thereby ensuring high quality crystallization while preventing mixed dispensing of dissimilar materials, contamination, etc.

Next, the stocker housing portion 3 will be described by referring to FIGS . 1 to 4. The illustrated stocker housing portion 3 is provided with the stocker robot 9, which supports a hand portion 8 detachably gripping microplates M, in such a manner that the handle portion can move back and forth, from side to side, up and down, and before and behind the passage for this lateral movement are arranged wide shelves 31, 32 to store the microplates. On the left and right end sides of the above-described stocker housing portion 3 are each provided a shelf 33, which temporarily houses and stores microplates M to be transferred to the crystal structural analysis process (hereinafter referred to as the analysis process), and a shelf 34 which houses suspension plates S, with the result that the microplates M and suspension plates S are housed in such a manner that the microplates M and suspension plates S can be mounted and dismounted from outside the stocker housing portion 3.

The rear-side shelf 32 is provided, within a prescribed width on the left-hand side thereof, with a supply set shelf 35 which supplies empty microplates M, and the above-described transit portion 3a is provided in the middle portion of left-hand side of the front-side shelf 31. Incidentally, each of the shelves is configured in such a manner that shelf portions which separately compartment and house microplates M and suspension plates S are packed.

The above-described transit portion 3a is configured so as to be able to perform transfer because microplates M can be mutually received and delivered between the hand portion 8 of the stocker robot 9 and a hand portion 55 of the stage robot 5. In this embodiment, this transit portion is in the form of a table (a transit table) which can bring microplates temporarily placed thereon into a standby condition so as to ensure efficient transit between the hand portion of the stocker robot and the hand portion of the stage robot. In the same way as described above, each individual operation, such as the transfer from the stage robot 5 to the side of the image processor 4 and taking out, transfer and housing by the stocker robot 9, is efficiently carried out by utilizing the transit waiting time with microplates M kept placed on the table.

And near this table is provided the marking portion 60, where plate name markings, such as plate numbers of microplates M and dates, and markings of selected test substances are indicated, for example, by barcode printing and marks which provide indicators.

And the transit portion 3a is provided, near the outside thereof, with the reading portion 61 (a plate name reader), such as a barcode reader, which reads marked plate names. This ensures that by utilizing the transit waiting time, the marking operation and reading operation are positively carried out during the mounting and dismounting of microplates M placed on the table of the transit portion 3a. And wrong transfer and wrong handling of various types of containers, etc. are prevented and the operation accuracy and efficiency are improved. The identification portion 6 is also provided in a compact manner within the installation space of the transit portion 3a.

Incidentally, in this embodiment, at least the stocker housing portion 3 is installed in a temperature-controlled room whose periphery is enclosed. The housing volume of the shelves 31, 32 is about 2500 microplates M and the supply set shelf 35 is formed to have a space capable of housing about 120 empty microplates M as a 1 day supply. On the supply set shelf 35, it is also possible by human being to freely supply and set empty microplates M, which are treated beforehand by the application of paraffin, from the outside of the stocker housing portion 3.

The above-described shelf 33 is of such a construction and a size that the shelf 33 can house about 40 microplates which contain appropriately crystallized test substances and can be transferred to the next analysis process. The operation of taking out and transferring the microplates to this analysis process can be carried out by human beings or automatically.

On the left end of the stocker housing portion 3, by installing the shelf 34 which can houses about 125 empty suspension plates S, the crystallization in microplates M under various conditions is efficiently performed. Furthermore, it is also possible to efficiently and easily grasp data on trial crystallization by use of various types of reagents.

The management system 1 formed in a temperature-controlled room is provided, for example, in a necessary place of the outer wall of the stocker housing portion, with an entrance and exit 3b that enables microplates M to be mounted and dismounted by human beings and various types of maintenance operation to be carried out.

Next, the stocker robot 9 installed in the stocker housing portion 3 will be described.

In this stocker robot 9, as shown in FIGS. 1 to 4, the hand portion 8 having a plane-face swing function and an advance and retreat function is backward and forward movably supported by a Z-axis actuating portion 90 in the form of a guide frame, which is horizontally installed in the backward and forward directions between the shelves 31, 32, and this Z-axis actuating portion 90 is supported by a Y-axis actuating portion 91 in columnar shape so as to be able to move up and down. The Y-axis actuating portion 91 is supported, at the top and bottom ends thereof, by X-axis actuating portions 92, 93 in rail shape laid on the floor surface and ceiling surface of the stocker housing portion 3 along the full length of the width so that the Y-axis actuating portion can move laterally. Furthermore, the hand portion 8 freely performs a horizontal swing motion by means of a swing actuating portion 95.

This construction enables the stocker robot 9 to take out microplates M and suspension plates S housed in the stoker housing portion 3 on the basis of commands from the control portion 7. Microplates M which have been taken out are automatically housed in the stocker housing portion 3 according to a set housing pattern. Also, the mounting and dismounting of microplates M by manual operation can be freely carried out.

Incidentally, a shelf portion which is partitioned so as to house one microplate M and one suspension plate S may be marked in such a manner that, for example, a section No. etc. of the shelf portion is indicated on the basis of shelf portions marked by the above-described marking portion 60. In this embodiment, however, markings are made for each microplate M supplied to the crystallization robot 2.

For microplates M containing test substances selected by the image processor 4, the positions of the selected test substances are indicated on these microplates M.

Next, the image processor 4 will be described. This image processor 4 is constituted by an image processing bed 40, a microscope portion (an image detection portion) 41, an image receiving and processing portion 42, a test-substance processing portion 71, etc. Microplates M, for which the dispensing operation by the crystallization robot 2 has been completed and crystallization proceeds during the temporary storage in the stocker housing portion 3, are supplied to the image processing bed 40 by the above-described stocker robot 9 and stage robot 5 after a lapse of a prescribed time or an arbitrary time. The microscope portion (the image detection portion) 41 is used to check the crystallization condition of the test substances in the microplates M placed on the image processing bed 40 for each test-substance well m by enlarging the test substances. The image receiving and processing portion 42 is used to receive and process checked images and send the checked images to the test-substance processing portion 71. This test-substance processing portion 71 displays images on a monitor screen on the basis of signals from the image receiving and processing portion 42, compares image data with data on test-substance image standards to make an evaluation (a judgment), and gives commands for storage places etc. of the relevant microplates M in the stocker housing portion 3 on the basis of the evaluation data.

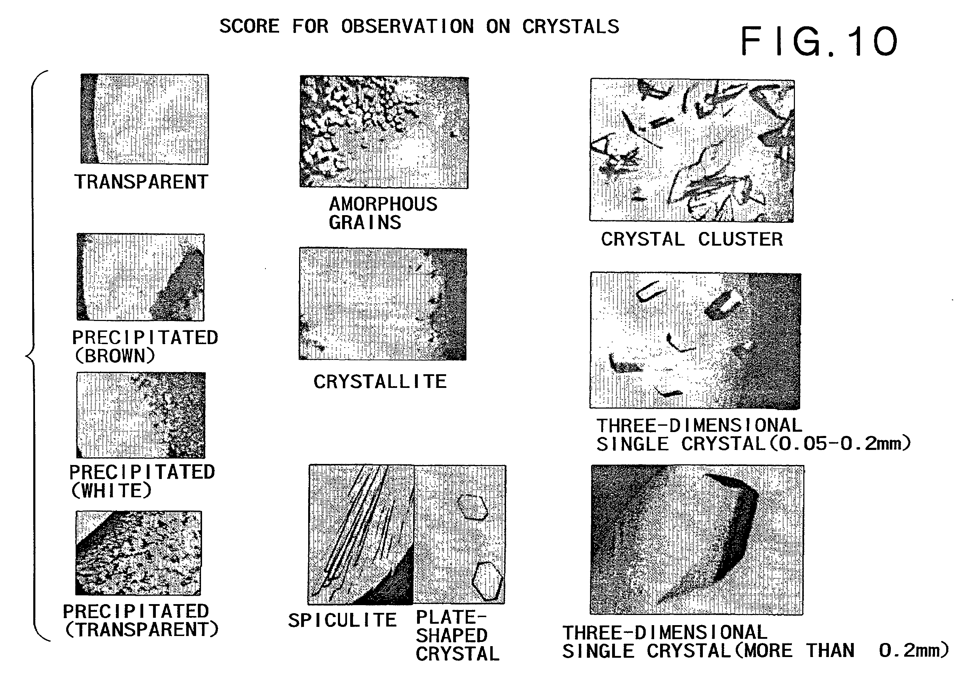

In the invention, standards for test-substance images (crystal image models) that provide a basis for the above-described data on test-substance image standards are set, as shown in FIG. 10, in the form of crystal image patterns divided into stages No. 1 to No. 9 or so. Among these image patterns, those of levels that permit a crystal structural analysis are set as standards for images of about 6 to 9 stages (hereinafter referred to as images permitting an analysis, and crystal image patterns that do not belong to the above-described images permitting an analysis are set as standards for images of about 1 to 5 stages.

That is, in setting the test-substance image standards as described above, for images not permitting an analysis, a test substance immediately after the dispensing operation falls under stage No. 0 in this example, because it is a transparent liquid. The test substance crystallizes gradually from a precipitation condition after a lapse of storage time and forms microcrystals while forming amorphous grains. When a test substance is in this region, about 1 to 5 stages are set for images not permitting an analysis. Furthermore, as preceding stages, a plurality of growth stages are set for images permitting an analysis in about 6 to 9 stages; they are the stage of formation of acicular crystals and plate crystals, the stage of formation of clusters of crystals after a lapse of time, and the stage of formation of three-dimensional single crystals. On the basis of the above-described patterning of stages of crystals, solving the problems of building, improving, etc. a database for the evaluation of crystallization drops and crystals is aimed at from the two aspects of hardware and software by the means which will be described later.

For the above-described test-substance image standards, the test-substance processing portion 71 recognizes each pattern of crystal image models as the number of pixels. Next, by use of the number of pixels of image data on each test substance in a microplate M detected by the image processor 4 as quantities of characteristics, the test-substance processing portion 71 automatically compares image data with data on test-substance image standards to make a judgment, and in the case of images which are in perfect or close agreement with the images permitting an analysis of any one of stages 6 to 9, the test-substance processing portion 71 causes the identification portion 6 to mark the positions of selected test substances on the microplate M. Subsequently, the test-substance processing portion 71 gives actuating commands to the stage robot 5 and the stocker robot 9, which house the microplate M in a designated shelf portion of the shelf 33.

In making a judgment as to whether a test substance in a microplate M has crystals permitting an analysis, this operation can be carried out not only automatically as described above, but also by human beings. In the case of the operation by human beings, it is also possible to capture an image by controlling the microscope portion 41 and to subject the data on captured image to various kinds of image processing in order to analyze quantities of characteristics related to the shapes and morphologies of crystallization drops and measured values etc. related to concentrations. Judgment results and image data obtained by these operations are stored in the database of a host computer installed outside and image processing and image control are carried out.

As a result of this, microplates M delivered from the crystallization robot 2 and stored in the stocker housing portion 3 are taken out one by one after a lapse of a predetermined time through the above-described transfer operation means and subjected to a check (a discrimination) to judge crystallization by the image processor 4. And at the same time, these microplates M are automatically transferred either to the re-storage process or the analysis process. In this manner, test substances are appropriately checked as to the degree of crystallization and microplates M which are re-stored are repeatedly checked many times. As a result, it is possible to prevent check omissions of test substances by overlooks and misidentifications, deterioration of crystals of test substances, and opportunity losses and waste, such as unnecessary re-checks, as observed in conventional crystallization methods and apparatuses, and high-accuracy, high-efficiency checking of test substances can be performed.

Furthermore, in the invention, microplates M containing test substances which are in agreement with the regions of images permitting an analysis in accordance with the test-substance image standards are collectively housed on the side of the shelf 33 through the above-described transfer means and, therefore, the transfer to the analysis process can be efficiently carried out. Moreover, the crystallization of test substances is promoted during the taking-out waiting time in this judgment process and, at the same time, by use of the transfer means and image processor 4, the relevant test substances and other test substances in microplates M are subjected to image processing in a concentrated manner to make a judgment. In addition, this information is input to the control portion 7 and supplied as reference information for the analysis process, whereby a crystal structural analysis is performed efficiently and with high accuracy.

Next, the stage robot will be described. In this stage robot 5, as shown in FIGS. 1 to 3, an X-axis actuating portion 50 is laterally installed in an apparatus frame 1a provided in a ceiling portion of a space, which is defined by the three portions of crystallization robot 2, stocker housing portion 3 and image processor 4, and this X-axis actuating portion 50 is provided so as to be able to laterally move Z-axis actuating portions 51, 51a and installed in the vertical direction. At the bottom end of the Z-axis actuating portion 51a, a Y-axis actuating portion 52, which performs plane-face swings around a longitudinal axis, is supported so as to be able to pivot, and at the leading end of the Y-axis arm portion is provided the hand portion 55, which detachably grips microplates M and suspension plates S.

As a result of this, on the basis of a sequence and timing commands set by the above-described tracking control portion 70 or the test-substance processing portion 71, the stage robot 5 operates in collaboration with the stocker robot 9 of stocker housing portion 3 and supplies and sets microplates M and suspension plates S taken out by the stocker robot 9 to the plate bed 20 of the crystallization robot 2 through the transit portion 3a.

The microplates M and suspension plates S on the plate bed 20 can be placed on the table of the transit portion 3a. The microplates M in the transit portion 3a are supplied to the image processing bed 40 of the image processor 4 and set thereon, and the microplates M on the image processing bed 40 are transferred to the transit portion 3a. These operations are repeatedly performed automatically on the basis of a set program, and arbitrary operations by human beings from the control portion 7 are also possible.

Furthermore, between the transit portion 3a and the image processor 4, the illustrated stage robot 5 is provided with a turntable 56 on which microplates M are temporarily placed and a transit posture to a next position is appropriately corrected. As a result of this, in a case where a microplate supplied to and housed in the stocker housing portion 3 is in a reverse direction, this fact is detected by the transit portion 3a etc., this microplate M is turned through about 180 degrees on the basis of this detection command and corrected to an appropriate posture, and the occurrence of troubles, such as a wrong judgment, in the preceding analysis process is prevented.

In this manner, the stage robot 5 performs the transit and delivery/receiving of microplates M to the crystallization robot 2 and stocker robot 9, whereby outside the stocker housing portion 3, by use of only one unit, it is possible to efficiently transfer microplates from the crystallization robot 2 to the stocker robot 9 and from the stocker robot 9 to the image processor 4 and besides the stocker robot 9, which is simple in construction, can be manufactured at low cost.

Incidentally, when a stage robot 5 as described above has a different configuration or the stocker housing portion can be miniaturized, the installation of the stage robot 5 may be omitted by providing the functions of the stage robot 5 in the stocker robot 9.

Next, by referring to FIGS. 2 and 9, the operation mode of the management system 1 configured as described above (operation of the crystallization control system) will be described below.

Incidentally, although names, numerical values, etc. are additionally written for reference on the arrow lines and each device in FIG. 9, the invention will not limited by this.

First, sample tubes T housing test substances are positioned and set by human operation on the plate bed 20 of the crystallization robot 2 from the front side in operation. Next, microplates M and suspension plates S housed on the side of the stocker housing portion 3 are set on the above-described plate bed 20 by transfer means constituted by the stocker robot 9 and the stage robot 5 and after that, the dispensing operation by the already described dispensing tube 21 is started.

At this time, while passing through the transit portion 3a, empty microplates M supplied to the crystallization robot 2 are marked one by one with sample numbers by the marking portion 60 of the identification portion 6 and distinguished from other microplates M.

In the dispensing operation, on the basis of operation procedures similar to conventional ones, a prescribed quantity of test substance is injected from a test-substance nozzle of the dispensing tube 21 into a test-substance well m of a microplate M. And after that, reagents are injected from a reagent nozzle into the relevant test-substance well m and both are mixed together.

After that, the nozzles of the dispensing tube 21 are cleaned by the cleaning unit U as required. Similar or dissimilar test substances (for example, Protein A, Protein B, ...) are injected into the next test-substance well m by the same dispensing means as described above, and diluted similar reagents or dissimilar reagents (for example, Reagent No. 1, Reagent No.2,...) are injected into this test-substance well m. The dispensing operation of all microplates M is completed by being repeated for all test-substancewells (about 72 wells).

At the same time with the completion of this dispensing operation, the microplates M as test-substance samples are taken out from the crystallization robot 2 by the hand portion 55 of the stage robot 5 and placed in the transit portion 3a, where after the reading of sample numbers by the reading portion 61 of the identification portion 6, the microplates M are gripped and received by the hand portion 8 of the stocker robot 9 and then transferred to and housed in specified empty shelf portions of the set selves 31, 35 of the stocker housing portion 3.

And while the above-described dispensing operation by the crystallization robot 2 and the housing operation by the stocker housing portion 3 are being sequentially repeated in prescribed quantities, the crystallization of the test substances in the microplates M proceeds gradually with the times. At this time, each test substance shows either crystals which meet the standards of the already described sample crystal patterns or uncompleted crystals. In principle, however, the stocker robot 9 takes out the microplates M one by one on the basis of the order of delivery to storage places and places the microplates M in the transit portion 3a. The stage robot 5 receives and transfers the microplates M, places the microplates M on the image processing bed 40 of the image processor 4, where image processing is performed by an image processing stage.

The image processor 4 causes a CCD camera of the image receiving and processing portion 42 to automatically photograph enlarged images (microscopic images) of the crystallization condition of the test substances in all test-substance wells m, sends the fetched image data to the test-substance processing portion 71 and an external memory (a host computer), where the data is stored. The external memory compares the fetched image data with the standard image data to make an evaluation.

In the invention, the progress stage of the crystal formation of the test substances are set as standard images in a plurality of stages by distinguishing between images in uncrystallized regions and images in crystal regions permitting an analysis. The data on the plurality of standard images and the fetched image data on the crystallization progress of the test substances are compared to make a judgment, and by selecting only test substances having fetched image data in perfect or close agreement with the above-described images of crystal regions permitting an analysis, containers M containing the test substances are transferred to the analysis process. Microplates M having evaluation data as the standard images data of the above-described No. 6 to No. 9 (i.e., test substances judged to be within the test-substance sample standard values) are automatically marked with markings indicating the selection and sequentially stored in the shelf 33 through the stage robot 5, transit portion 3a and stocker robot 9.

On the other hand, microplates M having evaluation data of No. 1 to No. 5 and hence substances which do not meet the test-substance standard values are housed again in the shelf portions of the shelves 31, 32 through the stage robot 5, transit portion 3a and stocker robot 9. A certain crystallization time is given to these microplates M and after that, the crystallization check operation by above-described crystallization stages is repeated. In this manner, crystallization, crystallization check and management operations are continuously carried out for all microplates M.

The crystallization of test substances in microplates M requires several hours to several days in early cases and a long period of 2 months or so in early cases. In the invention, it is possible to carry out the storage management of the crystallization of test substances for a long period without an error, with less labor and at low cost while automatically and sequentially performing the crystallization check operation of the test substances of all these microplates M by means of the image processor 4.

Furthermore, according to the image processing stage described above, in comparison with conventional processing by visual judgment by human beings using a microscope, which requires a time of about 30 minutes per microplate M (a 72-well microplate), the required time becomes about 4 minutes, which is not more than about 1/7 of the time required by conventional processing. Thus, the processing can be carried out in a very short time in a time-saving manner. Furthermore, the dispensing operation etc. by the crystallization robot 2 can be continued by setting a program of the tracking control portion 70 in this image processing time without causing the stage robot 5 and stocker robot 9 to be on standby uselessly and, therefore, the crystallization operation of test substances can be efficiently performed.

## Claims

1. A method for crystallization of proteins and the like, in which test substances prepared by mixing a sample of a protein and the like and reagents which promote the crystallization of the sample are put into test-substance wells of containers, such as microplates, and transferred to an analysis process, such as an X-ray crystal structural analysis, to perform an information analysis of the sample, comprising the steps of;
setting a plurality of standard images of progress stages of the test substances, such as crystal formation, as images in uncompleted crystallization regions and images in crystallization regions permitting an analysis;
comparing data on the plurality of standard images with fetched image data during the progress of crystallization of the test substances thereby to make a judgment;
selecting only test substances having fetched image data in perfect or close agreement with the images in crystallization regions permitting an analysis; and
transferring a container containing the test substances to the analysis process.

2. The method for crystallization of proteins and the like according to claim 1, wherein a mark to indicate the selection is automatically put to the container containing the test substances which are selected by comparing data on the plurality of standard images with the fetched image data to make a judgment.

3. The method for crystallization of proteins and the like according to claim 1, wherein the containers containing the test substances are stored on shelves constituting a stocker housing portion in such a manner as to permit the mounting and dismounting thereof and the stored containers are taken out one after another, and a selection of test substances is repeatedly performed by use of an image processor.

4. An apparatus for crystallization of proteins and the like, comprising:
a crystallization robot;
said crystallization robot preparing test substances by mixing a sample of a protein and the like and reagents which promote the crystallization of the sample in test-substance wells of containers such as microplates;
a stocker housing portion;
said stocker housing portion comprising a plurality of shelves which temporarily store the containers containing the test substances in such a manner as to permit the mounting and dismounting thereof;
an image processor;
said image processor performing image processing of the test samples in the containers stored in the stocker housing portion thereby to grasp the condition of crystallization;
a stocker robot;
said stocker robot performing the mounting and dismounting of the containers in the stocker housing portion; and
a stage robot;
said stage robot performs the transit of the containers from the stocker robot and the transfer and supply of the containers to the image processor.

5. The apparatus for crystallization of proteins and the like according to claim 4, wherein the apparatus is constructed in such a manner that the container containing test substances selected by the image processor for transfer to the analysis process is stored in the stocker housing portion.

6. The apparatus for crystallization of proteins and the like according to claim 4 or 5, wherein between the stocker robot which performs the mounting and dismounting of the containers in the stocker housing portion and the stage robot is provided a transit portion where the containers are temporarily placed and through this transit portion the transit of the containers between the stocker robot and the stage robot is performed.

7. The apparatus for crystallization of proteins and the like according to claim 4 or 5, wherein the apparatus is constructed in such a manner that the stage robot performs the transit of the containers containing the test substances from the crystallization robot side to the stocker robot side.
